Europäisches Patentamt

European Patent Office

Office européen des brevets

(19)

(11)     EP 0 892 634 B1

## (12)     FASCICULE DE BREVET EUROPEEN

(45) Date de publication et mention
de la délivrance du brevet:
**09.10.2002   Bulletin 2002/41**

(21) Numéro de dépôt: **97945918.7**

(22) Date de dépôt: **13.11.1997**

(51) Int Cl.7: **A61K 7/48**, A61K 7/06

(86) Numéro de dépôt international:
**PCT/FR97/02039**

(87) Numéro de publication internationale:
**WO 98/022081 (28.05.1998 Gazette 1998/21)**

(54) **UTILISATION DE L'OCTOXYGLYCERINE DANS UNE COMPOSITION COSMETIQUE ET/OU DERMATOLOGIQUE COMME ACTIF POUR LE TRAITEMENT DE LA SEBORRHEE ET/OU DE L'ACNE**

VERWENDUNG VON OKTOXYGLYZERIN IN EINER KOSMETISCHE UND/ODER DERMATOLOGISCHE ZUSAMMENSETZUNG ZUR BEHANDLUNG VON SEBORRHEA UND AKNE

USE OF OCTOXYGLYCERIN IN A COSMETIC AND/OR DERMATOLOGICAL COMPOSITION AS ACTIVE AGENT FOR THE TREATMENT OF SEBORRHOEA AND/OR ACNE

(84) Etats contractants désignés:
**DE ES FR GB IT**

(30) Priorité: **15.11.1996  FR 9613980**

(43) Date de publication de la demande:
**27.01.1999   Bulletin 1999/04**

(73) Titulaire: **L'OREAL**
**75008 Paris (FR)**

(72) Inventeurs:
• **CUPFERMAN, Sylvie**
**F-75015 Paris (FR)**
• **LAUGIER, Jean-Pierre**
**F-92160 Antony (FR)**

(74) Mandataire: **Tezier Herman, Béatrice**
**L'OREAL-DPI**
**6 rue Bertrand Sincholle**
**92585 Clichy Cédex (FR)**

(56) Documents cités:
**EP-A- 0 025 302        WO-A-93/25208**
**DE-A- 4 140 474**

• **DATABASE WPI Week 8108 Derwent Publications Ltd., London, GB; AN 81-12551d XP002036883 "Stable aq. powder dispersion cosmetic - obtd. by dispersing powder in gel obtd. by addn. of sucrose fatty acid ester, alpha monoglycerineether and surfactant to water" & JP 55 160 713 A (SHISEIDO) , 13 décembre 1980**

Remarques:
Le dossier contient des informations techniques présentées postérieurement au dépôt de la demande et ne figurant pas dans le présent fascicule.

EP 0 892 634 B1

**Description**

La présente invention a pour objet l'utilisation de l'octoxyglycérine dans une composition cosmétique ou dermatologique pour traiter la séborrhée et les désordres cutanés qui lui sont associés, notamment l'acné et/ou l'hyperséborrhée.

La sécrétion de sébum est un phénomène normal et utile à la peau comme à la chevelure. Cependant, l'hypersécrétion de sébum, appelée la séborrhée, entraîne des désagréments et parfois une pathologie cutanée, notamment une peau grasse et même acnéique, et par un état séborrhéique du cuir chevelu. L'hypersécrétion sébacée et la perturbation de la kératinisation du follicule pilo-sébacé ont pour conséquences l'obstruction du follicule pilo-sébacé et la formation de lésions rétentionnelles ou comédons.

Ces pathologies et en particulier l'acné et/ou l'hyperséborrhée, relèvent notamment de la colonisation de la peau ou du follicule pileux par des germes du genre *Propionibacterium* tels que *Propionibacterium acnés, Propionibacterium granulosum, Propionibacterium avidum.*

Ces germes indésirables métabolisent les triglycérides de sébum en libérant des acides gras irritants responsables de l'inflammation qui apparaît au cours de ces pathologies.

Pour lutter contre ces agents pathogènes, on utilise couramment des actifs tels que le triclosan, l'hexamidine, l'hexétidine et le chlorure de benzalkonium. Mais l'utilisation de ces actifs n'est pas dénuée d'effets secondaires. Ainsi, le triclosan présente une toxicité non négligeable, même par voie topique. En outre, il s'est révélé insuffisamment efficace, notamment dans certains véhicules où son activité est inhibée par les tensioactifs. L'hexamidine et l'hexétidine sous forme de sels sont des substances sensibilisantes, susceptibles de provoquer des allergies. Par ailleurs, le chlorure de benzalkonium peut se révéler irritant aux doses habituelles d'utilisation. De plus, il déstabilise des compositions contenant des tensioactifs anioniques.

On constate donc que subsiste le besoin d'actifs topiques ayant un effet sur les pathologies liées aux germes du genre *Propionibacterium*, ayant une action au moins aussi efficace que les composés de l'art antérieur, tout en ne présentant pas les inconvénients des composés connus.

La présente invention a justement pour objet l'utilisation de composés particuliers permettant d'obtenir cet effet.

La demanderesse a découvert que l'octoxyglycérine présentaient une forte activité sur *Propionibacterium acnes* et *Propionibacterium granulosum*, et pouvaient donc être utilisés dans une composition cosmétique ou dermatologique en tant qu'actif anti-séborrhéique et anti-acnéique.

Certes, le document DE-A-4140474 décrit l'utilisation de monoalkyléthers de glycérol dans des compositions de nettoyage et de soins de la peau. Toutefois, ces composés sont utilisés comme additifs surgraissants évitant le dessèchement de la peau dans des compositions antiseptiques et désinfectantes à base d'alcools, les monoalkyléthers de glycérol renforçant l'effet antiseptique de l'alcool tout en évitant le dessèchement de la peau. L'utilisation des monoalkyléthers de glycérol, particulièrement de l'octoxyglycérine, pour agir sur les pathologies liées à *Propionibacterium acnes* et *Propionibacterium granulosum,* et notamment pour traiter la séborrhée et l'acné, n'a jamais été décrite.

Le document WO 93/25208 décrit l'utilisation de monoalkyléthers de glycérol dans des compositions cytocides, antibactériens et spermicides. Ce document ne décrit pas spécifiquement l'activité des monoalkyléthers sur les germes du genre *Propionibacterium* et encore moins les pathologies liées à *Propionibacterium acnes* et *Propionibacterium granulosum,* et notamment la séborrhée et l'acné. De plus l'octoxyglycérine n'est pas cité dans ce document.

La présente invention a donc pour objet l'utilisation de l'octoxyglycérine dans une composition cosmétique pour traiter la séborrhée de la peau et/ou du cuir chevelu, et/ou les désordres cutanés associés à la séborrhée.

La présente invention a aussi pour objet l'utilisation de l'octoxyglycérine pour la fabrication d'un médicament destiné à traiter la séborrhée de la peau et/ou du cuir chevelu, et/ou les désordres cutanés associés à la séborrhée.

Les désordres cutanés associés à la séborrhée peuvent notamment être l'acné, la peau grasse à tendance acnéique et/ou l'hyperséborrhée.

Aussi, la présente invention a encore pour objet l'utilisation de l'octoxyglycérine dans une composition cosmétique pour traiter l'acné et/ou la peau grasse à tendance acnéique et/ou l'hyperséborrhée.

La présente invention a encore pour objet l'utilisation de l'octoxyglycérine pour la fabrication d'un médicament destiné à traiter l'acné et/ou la peau grasse à tendance acnéique et/ou l'hyperséborrhée.

La présente invention a encore pour objet l'utilisation de l'octoxyglycérine dans une composition cosmétique pour agir sur les germes du genre *Propionibacterium*.

La présente invention a encore pour objet l'utilisation de l'octoxyglycérine pour la fabrication d'un médicament destiné à agir sur les germes du genre *Propionibacterium.*

La présente invention a aussi pour objet un procédé de traitement cosmétique de la séborrhée et/ou des désordres associés à la séborrhée, consistant à appliquer sur la peau et/ou le cuir chevelu présentant des troubles de la séborrhée, une composition contenant au moins de l'octoxyglycérine.

La présente invention a également pour objet un procédé de traitement cosmétique de l'acné consistant à appliquer sur la peau acnéique une composition contenant au moins de l'octoxyglycérine.

Enfin, l'invention a pour objet une composition cosmétique et/ou dermatologique anti-acnéique, caractérisée en ce qu'elle contient au moins, dans un milieu cosmétiquement et/ou dermatologiquement acceptable, une quantité efficace d'octoxyglycérine à titre d'actif anti-acnéique.

L'octoxyglycérine, (1,(2-éthylhexyl)glycéroléther), répond à la formule (I) suivante :

$$R\text{-}O\text{-}CH_2\text{-}CHOH\text{-}CH_2OH \tag{I}$$

dans laquelle R représente un radical 2-éthylhexyl.

L'octoxyglycérine est utilisé, selon la présente invention, en une quantité allant de préférence de 0,05 à 10 %, et mieux de 0,1 à 5 %, et encore mieux de 0,1 à 2.5 % du poids total de la composition.

Les compositions selon l'invention contiennent un milieu cosmétiquement et/ou dermatologiquement acceptable, c'est-à-dire compatible avec la peau, les muqueuses, les cheveux et le cuir chevelu. Elles peuvent se présenter sous toutes les formes galéniques appropriées pour une application topique, notamment sous forme de solutions aqueuses, hydroalcooliques ou huileuses, de gels aqueux, hydroalcooliques ou huileux, de produits anhydres pâteux ou solides, d'émulsions obtenues par dispersion d'une phase grasse dans une phase aqueuse (H/E) ou -inversement (E/H), de suspensions, de microémulsions, de microcapsules, de microparticules, ou encore de dispersions vésiculaires de type ionique (liposomes) et/ou non ionique. Ces compositions sont préparées selon les méthodes usuelles dans les domaines considérés.

Les compositions selon l'invention peuvent être également utilisées sous forme de mousses ou encore sous forme de compositions pour aérosol contenant également un agent propulseur sous pression.

Les quantités des différents constituants des compositions selon l'invention sont celles classiquement utilisées dans les domaines considérés.

Ces compositions constituent notamment des produits pour la peau tels que des gels ou crèmes de nettoyage ou de traitement, des lotions ou laits purifiants, des sticks camouflants, des lotions pour les cheveux ou des shampooings antiséborrhéiques, des compositions antichute pour le cuir chevelu.

Les compositions de l'invention peuvent comprendre les adjuvants classiquement mis en oeuvre dans les domaines considérés comme les corps gras, les solvants organiques, les agents solubilisants, les agents épaississants et gélifiants, les adoucissants, les antioxydants, les opacifiants, les agents stabilisants, tes agents moussants, les parfums, les émulsionnants ioniques ou non, les charges, les séquestrants et les chélateurs, les conservateurs, les parfums, les filtres, les huiles essentielles, les matières colorantes, les pigments, les agents actifs hydrophiles ou lipophiles, les vésicules lipidiques ou tout autre ingrédient habituellement utilisé en cosmétique. Ces adjuvants sont introduits dans les quantités habituellement utilisées dans le domaine cosmétique ou dermatologique, et par exemple de 0 à 20 % du poids total de la composition.

Lorsque la- composition de l'invention est une émulsion, la proportion de la phase grasse peut aller de 5 à 80 % et de préférence de 5 à 50 % du poids total de la composition. Les huiles, les émulsionnants et les coémulsionnants utilisés dans la composition sous forme d'émulsion sont choisis parmi ceux classiquement utilisés dans le domaine cosmétique ou dermatologique. L'émulsionnant et éventuellement le coémulsionnant sont présents, dans la composition, en une proportion allant de 0,3 à 30 % en poids, et de préférence de 0,5 à 20 % en poids par rapport au poids total de la composition. L'émulsion peut, en outre, contenir des vésicules lipidiques.

Comme huiles utilisables dans l'invention, on peut citer les huiles minérales (isoparaffine), les huiles d'origine végétale (huile d'amandes d'abricot), les huiles d'origine animale, les huiles de synthèse, les huiles siliconées (cyclopentadiméthylsiloxane) et les huiles fluorées. On peut aussi utiliser comme matières grasses des alcools gras, des acides gras (acide stéarique), des cires (cire de carnauba, ozokérite).

Comme émulsionnants utilisables dans l'invention, on peut citer par exemple le monostéarate d'éthylène glycol, le tristéarate de sorbitane, le mélange palmitostéarate de glycol / stéarate de polyéthylène glycol (6 OE) / stéarate de polyéthylène glycol (32 OE) vendu sous la dénomination « Tefose 63 » par la société Gattefosse, et la lécithine hydrogénée.

Comme gélifiants ou épaississants, on peut citer les gommes naturelles (gomme de xanthane), les polysaccharides (hydroxypropylméthylcellulose, carboxyméthylcellulose), les polymères carboxyvinyliques (carbomer), les copolymères acryliques, les polyacrylamides et notamment le mélange de polyacrylamide, $C_{13}$-$C_{14}$-Isoparaffine et Laureth-7, vendu sous la dénomination de Sepigel 305 par la société Seppic, les dérivés de sucres oxyéthylénés tels que le méthylglucose oxyéthyléné.

Comme agents moussants, on peut citer par exemple le N-carboxyéthoxyéthyl N-cocoylamidoéthyl aminoacétate N-di-sodique, le lauryl éther sulfate de sodium, le lauroyl sarcosinate de sodium, le triéthanolamine lauryl sulfate et encore le mélange de cocoyl isethionate de sodium et d'acides gras de coprah.

Comme charges, on peut citer notamment les copolymères acryliques tels que le copolymère diméthacrylate d'éthylène glycol/ methacrylate de lauryle vendu par la société Dow Corning sous la dénomination de Polytrap.

Comme agents actifs hydrophiles ou lipophiles, on peut citer notamment les agents susceptibles de compléter l'effet de l'alkyléther de glycérol dans le traitement de la séborrhée et des dermatoses associées, et notamment de l'acné. Il peut s'agir par exemple d'agents anti-inflammatoires tels que le peroxyde de benzoyle, d'antibiotiques, d'agents antiseptiques tels que l'octopirox, d'agents kératolytiques tels que les α-hydroxy-acides ou les β-hydroxy-acides sous forme libre, éthérifiée ou estérifiée, l'acide rétinoïque et ses dérivés, le rétinol et ses dérivés tels que le palmitate, l'acétate ou le propionate de rétinol, d'agents antiséborrhéiques tels que les sels de métaux di- ou trivalents comme les sels des métaux alcalino-terreux et les lanthanides, et plus particulièrement les sels de strontium et les sels de néodyme. Selon un mode préféré de réalisation de l'invention, cet agent est un agent kératolytique, et notamment un α-hydroxy-acide ou un β-hydroxy-acide.

La quantité d'agent peut aller par exemple de 0 à 20 % et de préférence de 0,05 à 5 % du poids total de la composition.

Comme α-hydroxyacides, on peut citer en particulier les acides glycolique, lactique, malique, tartrique, citrique, mandélique. Comme β-hydroxyacides, on peut citer l'acide salicylique ainsi que ses dérivés, les acides hydroxy-2 alcanoïques et leurs dérivés comme l'acide hydroxy-2-méthyl-3-benzoïque et l'acide hydroxy-2-méthoxy-3-benzoïque.

Par ailleurs, comme agents actifs hydrophiles, on peut citer aussi par exemple les protéines ou les hydrolysats de protéine, les acides aminés, les polyols (glycérine, propylène glycol), l'urée, l'allantoïne, les sucres et les dérivés de sucre, les vitamines, l'amidon, les extraits bactériens ou végétaux.

Lorsque la peau comportant les désordres cutanés liés à l'acné ou la séborrhée doit être exposée au soleil, la composition contenant l'alkyléther contient en plus, de manière appropriée, au moins un filtre, de façon à préserver l'efficacité de l'actif selon l'invention tout en protégeant la peau des effets néfastes des rayons solaires. Comme filtres, on peut citer les filtres organiques ou les pigments qui peuvent être sous forme de nanoparticules (nanopigments) ou non. Des exemples de filtres organiques sont les dérivés sulfonés ou sulfonatés de la benzophénone, les dérivés sulfoniques ou sulfonatés du benzylidène camphre et les acrylates tels que l'octocrylène. La quantité de filtre dépend de la protection solaire souhaitée. Elle peut aller par exemple de 0 à 10 % et de préférence de 0,1 à 5 % du poids total de la composition.

Les exemples suivants illustrent l'invention sans en limiter aucunement la portée. Les pourcentages sont donnés en poids.

**Exemple 1 : Gel moussant pour peaux grasses séborrhéiques**

-   Copolymère d'alcool de suif hydrogéné oxyéthyléné (60 OE) /myristyl glycol (solubilisant) (Elfacos GT 282 S vendu par la société Akzo)            0,9 %
-   Octoxyglycérine            0,1 %
-   Glycérine            3 %
-   Acide glycolique            1 %
-   N-carboxyéthoxyéthyl N-cocoylamidoéthyl aminoacétate N-di-sodique à 38 % dans l'eau            5 %
-   Lauryl éther sulfate de sodium            14,3 %
-   Diéthanolamide d'acides gras de coprah (adoucissant)   0,7 %
-   Eau déminéralisée            qsp 100 %

Mode opératoire :

On disperse dans de l'eau déminéralisée, à 80°C, l'Elfacos GT 282S, puis on refroidit à 50°C. Par ailleurs, on disperse à 50°C dans de l'eau déminéralisée, les agents moussants, l'adoucissant et la glycérine sous agitation lente, puis on verse doucement cette préparation sous agitation lente dans la dispersion d'Elfacos GT 282S. On y ajoute ensuite à 40°C l'octoxyglycérine.

Le gel obtenu convient pour le traitement de la dermite séborrhéique, en application deux fois par jour sur le visage.

EP 0 892 634 B1

**Exemple 2 : Crème nettoyante moussante pour peaux acnéiques**

- Monostéarate d'éthylène glycol 2 %
- Octoxyglycérine 2,5 %
- Silicate de magnésium et d'aluminium hydraté (stabilisant) 1,7 %
- hydroxypropylméthylcellulose 0,8 %
- Mélange de cocoyl isethionate de sodium et d'acides gras de coprah (Elfan AT 84 G vendu par la société Akzo) 15 %
- Acide stéarique 1,25 %
- Lauroyl sarcosinate de sodium à 30 % dans l'eau 10 %
- Parfum 0,3 %
- Eau déminéralisée qsp 100 %

Mode opératoire :

On disperse l'Elfan AT 84 G dans de l'eau déminéralisée à 80°C. Après dissolution complète, on ajoute le sel de silicate de magnésium et d'aluminium hydraté. On refroidit à 60°C, puis on ajoute le lauroyl sarcosinate de sodium et le gel hydroxypropylméthylcellulose. On ajoute ensuite l'acide stéarique et le monostéarate d'éthylène glycol préalablement réchauffés au bain-marie. A ce mélange, on additionne l'octoxyglycérine et le parfum:
La crème obtenue convient pour le traitement de la peau acnéique, par application sur le visage deux fois par jour.

**Exemple 3 : Crème traitante pour l'acné**

- Tristéarate de sorbitane 1 %
- Octoxyglycérine 0,5 %
- Carbomer 0,4 %
- Gomme de xanthane 0,5 %
- Polytrap (Dow Corning) 1 %
- Cyclopentadiméthylsiloxane (huile volatile) 6 %
- Glycérine 3 %
- Tefose 63 (Gattefosse) (émulsionnant) 4 %
- Eau déminéralisée qsp 100 %

Mode opératoire :

On prépare le mélange A contenant le tristéarate de sorbitane, l'émulsionnant et le cyclopentadiméthylsiloxane à 60°C. Par ailleurs, on prépare le mélange B en faisant gonfler, dans l'eau déminéralisée à 70°C, le mélange de glycérol et de carbomer, puis la gomme de xanthane. On réalise l'émulsion à 65° C en versant le mélange A dans le mélange B sous agitation. On laisse refroidir à 50°C et on ajoute le Polytrap, puis l'octoxyglycérine.
La crème obtenue convient pour le traitement de la peau, par application sur le visage et le dos du corps une fois par jour.

**Exemple 4 : Gel traitant pour peaux séborrhéiques**

- Octoxyglycérine 5 %
- Gomme de xanthane 1 %
- Glycérine 2 %
- Parfum 0,2 %
- Alcool éthylique (solvant) 30 %
- Mélange alcool butylique oxyéthyléné (26 OE) - oxypropyléné (26 OP) / huile de ricin hydrogénée oxyéthylénée (40 OE) dans l'eau (Solubilisant LRI vendu par la société Wackherr) (co-solvant) 1 %

- Eau déminéralisée       qsp 100 %

Mode opératoire :

On fait gonfler la gomme de xanthane dans l'eau déminéralisée avec la glycérine à 70°C. Après refroidissement à 30°C, on ajoute l'acide glycolique, l'alcool éthylique, le co-solvant avec l'octoxyglycérine et le parfum.
Le gel obtenu convient pour le traitement de la peau séborrhéique, en application une à deux fois par jour sur le visage.

**Exemple 5 : Crème teintée traitante pour peaux acnéiques, contenant des liposomes**

- Stérols de soja oxyéthylénés (5 OE)      1,6 %
- Lécithine hydrogénée      2,4 %
- Octoxyglycérine      2 %
- Huile d'amandes d'abricot      6 %
- Oxyde de fer jaune (pigments)      0,45 %
- Oxydes de fer brun (pigments)      0,4 %
- Oxyde de fer noir (pigments)      0,07 %
- Oxyde de titane (pigments)      5 %
- Polytrap (Dow Corning)      1 %
- polyacrylamide / $C_{13}$-$C_{14}$-Isoparaffine / Laureth-7 (Sepigel 305)    4 %
- Cyclopentadiméthylsiloxane (huile volatile)    6 %
- Glycérine      6 %
- Propylène glycol      6 %
- Eau déminéralisée      qsp 100 %

Mode opératoire :

Pour préparer les liposomes, on hydrate le mélange de stérols de soja oxyéthylénés (5 OE) et de lécithine hydrogénée dans de l'eau déminéralisée à 80°C pendant 2 heures et on passe deux fois cette préparation à l'homogénéisateur haute pression. Après avoir refroidi à 35°C la préparation de -liposomes, on y ajoute l'huile d'amandes d'abricot et le cyclopentadiméthylsiloxane. On passe le mélange à l'homogénéisateur haute pression et on refroidit à 30°C. Par ailleurs, on prépare une dispersion des charges dans le propylène glycol et le glycérol. On disperse cette dispersion dans le mélange obtenu précédemment. On ajoute ensuite le Sepigel 305 et l'octoxyglycérine.
La crème obtenue est de couleur beige et elle convient pour le traitement de la peau, par application sur le visage deux fois par jour.

**Exemple 6 : Stick camouflant pour peaux grasses**

- Octoxyglycérine      1 %
- Cire de carnauba      8 %
- Ozokérite      6 %
- Oxyde de fer jaune (pigments)      2,5 %
- Oxydes de fer brun (pigments)      1 %
- Oxyde de fer noir (pigments)      0,5 %
- Oxyde de titane (pigments)      20 %
- Polydiméthylsiloxane / silice hydratée (agent de démoulage)    0,1 %
- Isoparaffine      qsp 100 %

Mode opératoire :

On disperse dans le mélange de cires et d'huile, à 100°C, les oxydes et l'agent de démoulage, pendant 2 heures. On y ajoute l'octoxyglycérine juste avant le coulage.
Le stick obtenu peut être appliqué sur le visage plusieurs fois par jour.

**Exemple 7 : Lotion purifiante pour peaux acnéiques**

- Octoxyglycérine      2 %
- Glycérine      2 %
- Alcool éthylique (solvant)      30 %
- Alcool butylique oxyéthyléné (26 OE) oxypropyléné (26 OP) / huile de ricin hydrogénée oxyéthylénée (40 OE) dans l'eau(Solubilisant LRI vendu par la société Wackherr) (co-solvant)      1 %
- Eau déminéralisée      qsp 100 %

Mode opératoire :

On mélange dans l'eau déminéralisée, la glycérine, l'alcool éthylique, le co-solvant et l'octoxyglycérine.

La lotion obtenue convient pour le nettoyage de la peau, par application sur le visage deux fois par jour.

**Exempte 8 : Shampooing antiséborrhéique**

- Octoxyglycérine      2 %
- Triéthanolamine lauryl sulfate à 40 % dans l'eau      37 %
- Diéthanolamide d'acides gras de coprah (agent conditionneur de cheveux)      2 %
- Carboxyméthylcellulose      0,5 %
- Méthylglucose oxyéthyléné      2,5 %
- Eau déminéralisée      qsp 100 %

Mode opératoire :

On disperse dans l'eau déminéralisée la carboxyméthylcellulose, et on ajoute l'agent conditionneur, le triéthanolamine lauryl sulfate et le méthylglucose oxyéthyléné. Après avoir refroidi à 40°C, on ajoute l'octoxyglycérine.

Le shampooing obtenu convient pour le lavage des cheveux et permet une nette amélioration de l'état du cuir chevelu.

**Test:**

Le test décrit ci-dessous met en évidence l'activité de l'octoxyglycérine sur *Propionibacterium acnes* et *Propionibacterium granulosum.*

Les étapes pour réaliser ce test sont les suivantes :

1) Culture du microorganisme : *Propionibacterium acnes* et *Propionibacterium granulosum* sont cultivés sur gélose en pente Tryptocaséine soja

2) Préparation de inoculum : 48 heures avant le début du test, on effectue un repiquage de la souche et on laisse incuber pendant 48 heures à 35°C en conditions d'anaérobiose. La suspension obtenue titre à $10^8$ germes/ml.

3) Préparation de l'échantillon : On dépose dans un flacon de verre appelé pilulier, 32 g d'un bouillon Tryptocaséine soja et on laisse incuber à 35°C pendant 24 heures. Puis, on ajoute 4 g de la composition à tester et on homogénéise. Parallèlement, on prépare un témoin pour vérifier que les germes sont dans des conditions de croissance favorables pendant la durée du test.

4) Inoculation : on introduit 4 ml d'inoculum dans le pilulier et on place le pilulier dans l'incubateur.

5) Prélèvements et dénombrements : après chaque temps de contact (2, 4, 6 et 24 heures), on homogénéise le contenu du pilulier, et on en prélève 1 ml. Après avoir déterminé la dilution appropriée pour pouvoir effectuer le comptage, on étale cette dilution en surface de boîtes de Pétri gélosées (milieu Eugon LT100), puis on laisse incuber les boîtes de Pétri pendant 7 jours à l'étuve à 35°C en conditions d'anaérobiose.

Puis, on effectue le comptage des colonies sur les boîtes contenant plus de 20 et moins de 200 colonies.

Les compositions testées sont les suivantes :

|  | Préparation A | Préparation B |
|---|---|---|
| Eau distillée stérile | 57,99 g | 59,99 g |
| Propylène glycol | 20 g | 20 g |
| Alcool éthylique | 20 g | 20 g |
| Acide lactique | 0,01 g | 0,01 |
| Octoxyglycérine | 2 g | ----- |

Les résultats obtenus sont indiqués dans le tableau suivant. Ils sont exprimés en nombre de micro-organismes par gramme de préparation :

| | Propionibacterium acnes | | Propionibacterium granulosum | |
|---|---|---|---|---|
| Temps de contact | Préparation A | Préparation B | Préparation A | Préparation B |
| 0 | $3,4.10^7$ | $3,4.10^7$ | $3,6.10^7$ | $3,6.10^7$ |
| 2 | $1,7.10^4$ | $6,8.10^7$ | $7,5.10^3$ | $4,5.10^7$ |
| 4 | $5,2.10^9$ | $7,9.10^7$ | < 200* | $4,6.10^7$ |
| 6 | $5,2.10^3$ | $9,4.10^7$ | < 200* | $4,3.10^7$ |
| 24 | < 200* | $1,9.10^8$ | < 200* | $3,7.10^8$ |

*Seuil de sensibilité de la méthode

On constate dès 2 heures de contact, une décontamination supérieure avec la préparation A selon l'invention. Le nombre de micro-organismes est 1000 fois inférieur pour *Propionibacterium acnes* et 10000 fois inférieur pour *Propionibacterium granulosum* au taux d'inoculation (T0) alors que pour la préparation B, il est inchangé.

Ce résultat est confirmé et accentué à 24 heures de contact puisque le nombre de micro-organismes revivifiables est inférieur au seuil de détection de la méthode pour la préparation A, alors que, dans le cas de la préparation B, il est environ dix fois supérieur au taux d'inoculation (T0).

**Revendications**

1. Utilisation de l'octoxyglycérine dans une composition cosmétique pour lutter contre les germes du genre *Propionibacterium*.

2. Utilisation de l'octoxyglycérine pour la fabrication d'un médicament destiné à lutter contre les germes du genre *Propionibacterium*.

3. Utilisation selon l'une quelconque des revendications 1 ou 2, pour traiter la séborrhée de la peau et/ou du cuir chevelu, et/ou les désordres cutanés associés à la séborrhée.

4. Utilisation selon l'une quelconque des revendications 1 à 3, pour traiter l'acné et/ou la peau grasse à tendance acnéique et/ou l'hyperséborrhée.

5. Utilisation selon l'une quelconque des revendications précédentes, **caractérisée en ce que** de l'octoxyglycérine est présente en une quantité allant de 0,05 à 10 % en poids de la composition.

6. Utilisation selon la revendication précédente, **caractérisée en ce que** de l'octoxyglycérine est présente en une quantité allant de 0,1 à 2,5 % en poids de la composition

7. Utilisation selon l'une quelconque des revendications précédentes, **caractérisée en ce que** la composition est une solution aqueuse, huileuse ou hydroalcoolique, une émulsion, une microémulsion, un gel aqueux, huileux ou anhydre, un sérum, une dispersion de vésicules, un produit anhydre solide ou pâteux.

8. Utilisation selon l'une quelconque des revendications précédentes, **caractérisée en ce que** la composition se présente sous forme d'une crème, d'une lotion, d'un gel ou d'un stick.

9. Utilisation selon l'une quelconque des revendications précédentes, **caractérisée en ce que** la composition comprend au moins un adjuvant choisi parmi les corps gras, les solvants organiques, les agents solubilisants, les gélifiants, les agents moussants, les émulsionnants, les charges, les filtres, les pigments, les agents actifs hydrophiles ou lipophiles, les vésicules lipidiques.

10. Utilisation selon l'une quelconque des revendications précédentes, **caractérisée en ce que** la composition comprend au moins un agent choisi parmi les $\alpha$-hydroxyacides, les $\beta$-hydroxyacides et les sels des métaux di- ou trivalents.

11. Procédé de traitement cosmétique de la séborrhée et/ou des désordres associés à la séborrhée, consistant à appliquer sur la peau et/ou le cuir chevelu présentant des troubles de la séborrhée, une composition contenant au moins de l'octoxyglycérine.

12. Procédé de traitement cosmétique de l'acné, consistant à appliquer sur la peau.acnéique une composition contenant au moins de l'octoxyglycérine.

**Patentansprüche**

1. Verwendung von Octoxyglycerin in einer kosmetischen Zusammensetzung zur Bekämpfung von Keimen der Gattung *Propionibacterium.*

2. Verwendung von Octoxyglycerin zur Herstellung eines Arzneimittels, das zur Bekämpfung von Keimen der Gattung *Propionibacterium* bestimmt ist.

3. Verwendung nach einem der Ansprüche 1 oder 2 zur Behandlung der Seborrhoe der Haut und/oder der Kopfhaut und/oder von mit Seborrhoe verbundenen Hautstörungen.

4. Verwendung nach einem der Ansprüche 1 bis 3 zur Behandlung von Akne und/oder fettiger Haut mit Tendenz zu Akne und/oder Hyperseborrhoe.

5. Verwendung nach einem der vorhergehenden Ansprüche, **dadurch gekennzeichnet, dass** Octoxyglycerin in einem Mengenanteil von 0,05 bis 10 Gew.-% der Zusammensetzung vorliegt.

6. Verwendung nach dem vorhergehenden Anspruch, **dadurch gekennzeichnet, dass** Octoxyglycerin in einem Mengenanteil von 0,1 bis 2,5 Gew.-% der Zusammensetzung vorliegt.

7. Verwendung nach einem der vorhergehenden Ansprüche, **dadurch gekennzeichnet, dass** es sich bei der Zusammensetzung um eine wässerige, ölige oder wässerig-alkoholische Lösung, eine Emulsion, eine Mikroemulsion, ein wässeriges, öliges oder wasserfreies Gel, ein Serum, eine Vesikeldispersion oder um ein festes oder pastöses wasserfreies Produkt handelt.

8. Verwendung nach einem der vorhergehenden Ansprüche, **dadurch gekennzeichnet, dass** die Zusammensetzung in Form einer Creme, einer Lotion, eines Gels oder eines Stifts vorliegt.

9. Verwendung nach einem der vorhergehenden Ansprüche, **dadurch gekennzeichnet, dass** die Zusammensetzung mindestens einen Zusatzstoff enthält, der unter Fettsubstanzen, organischen Lösemitteln, Lösungsvermittlern, Gelbildnern, Schaumbildnern, Emulgatoren, Füllstoffen, Filtern, Pigmenten, hydrophilen oder lipophilen Wirkstoffen und Lipidvesikeln ausgewählt ist.

10. Verwendung nach einem der vorhergehenden Ansprüche, **dadurch gekennzeichnet, dass** die Zusammensetzung mindestens einen Wirkstoff enthält, der unter $\alpha$-Hydroxysäuren, $\beta$-Hydroxysäuren und den Salzen von zweiwertigen oder dreiwertigen Metallen ausgewählt ist.

11. Verfahren zur kosmetischen Behandlung von Seborrhoe und/oder mit Seborrhoe assoziierten Störungen, das darin besteht, eine Zusammensetzung, die zumindest Octoxyglycerin enthält, auf die Haut und/oder die Kopfhaut, die seborrhoische Störungen aufweist, aufzubringen.

12. Verfahren zur kosmetischen Behandlung von Akne, das darin besteht, eine Zusammensetzung, die zumindest Octoxyglycerin enthält, auf die von Akne befallene Haut aufzubringen.

**Claims**

1. Use of octoxyglycerol in a cosmetic composition for combatting microorganisms of the genus *Propionibacterium*.

2. Use of octoxyglycerol in the manufacture of a medicament intended to combat microorganisms of the genus *Propionibacterium*.

3. Use according to either one of Claims 1 and 2 for treating seborrhoea of the skin and/or of the scalp and/or cutaneous disorders associated with seborrhoea.

4. Use according to any one of Claims 1 to 3 for treating acne and/or greasy skin with a tendency towards acne and/or hyperseborrhoea.

5. Use according to any one of the preceding claims, **characterized in that** octoxyglycerol is present in an amount ranging from 0.05 to 10% by weight of the composition.

6. Use according to the preceding claim, **characterized in that** octoxyglycerol is present in an amount ranging from 0.1 to 2.5% by weight of the composition.

7. Use according to any one of the preceding claims, **characterized in that** the composition is an aqueous, oily or aqueous/alcoholic solution, an emulsion, a microemulsion, an aqueous, oily or anhydrous gel, a serum, a dispersion of vesicles or a pasty or solid anhydrous product.

8. Use according to any one of the preceding claims, **characterized in that** the composition is provided in the form of a cream, of a lotion, of a gel or of a stick.

9. Use according to any one of the preceding claims, **characterized in that** the composition comprises at least one adjuvant chosen from fatty substances, organic solvents, solubilizing agents, gelling agents, foaming agents, emulsifiers, fillers, screening agents, pigments, hydrophilic or lipophilic active agents, or lipid vesicles.

10. Use according to any one of the preceding claims, **characterized in that** the composition comprises at least one agent chosen from α-hydroxyacids, β-hydroxyacids and salts of di- or trivalent metals.

11. Process for the cosmetic treatment of seborrhoea and/or of disorders associated with seborrhoea, which consists in applying a composition containing at least octoxyglycerol on the skin and/or the scalp exhibiting disorders of seborrhoea.

12. Process for the cosmetic treatment of acne, which consists in applying, on skin suffering from acne, a composition containing at least octoxyglycerol.